Europäisches Patentamt

European Patent Office   (11) Publication number:   **0 154 481**

Office européen des brevets   **B1**

# EUROPEAN PATENT SPECIFICATION

(45) Date of publication of patent specification: **08.02.89**   (51) Int. Cl.⁴: **C 07 C 43/253,** C 07 C 41/01, C 07 C 41/03

(21) Application number: **85301249.0**

(22) Date of filing: **25.02.85**

(54) Process for making substituted phenoxycycloalkanols.

(30) Priority: **24.02.84 US 583452**

(43) Date of publication of application:
**11.09.85 Bulletin 85/37**

(45) Publication of the grant of the patent:
**08.02.89 Bulletin 89/06**

(84) Designated Contracting States:
**AT CH DE FR GB IT LI NL SE**

(56) References cited:
**US-A-3 272 854**
**US-A-4 249 024**

**(Houben-Weyl) E. Müller "Methoden der organischen Chemie", VI/3, 1965, pp. 43,79**

**The file contains technical information submitted after the application was filed and not included in this specification**

(73) Proprietor: **UNIROYAL CHEMICAL COMPANY, Inc.**
**World Headquarters**
**Middlebury Connecticut 06749 (US)**

(72) Inventor: **Ma, Shih Y.**
**822 Roaring Brook Road Cheshire**
**New Haven Connecticut 06410 (US)**
Inventor: **Covery, Rupert A.**
**112 Lebanon Road Bethany**
**New Haven Connecticut 06525 (US)**

(74) Representative: **Harrison, Michael Robert et al**
**URQUHART-DYKES & LORD 5th Floor Tower House Merrion Way**
**Leeds LS2 8PA West Yorkshire (GB)**

EP 0 154 481 B1

## Description

### Background of the disclosure
### 1. Field of the invention

The instant invention is directed to a process for making substituted phenoxycycloalkanols by reacting a cycloakylene oxide with a sodium containing compound as defined in the claims.

### 2. Background of the prior art

Cycloaliphatic sulfite esters have found wide acceptance as insecticides and, more particularly, as miticides. In addition, these compounds are used as plasticizers. A class of intermediate compounds useful in the manufacture of these ester compounds are the phenoxycycloalkanols. Such intermediate compunds are disclosed as useful in the manufacture of cycloaliphatic sulfite esters in U.S. Patent 3,272,854 issued by Covey et al.

U.S. Patent 3,272,854 discloses a method of making such a compound by reacting p-tert-butylphenol with cyclohexene oxide in the presence of 1 to 1.5% by weight, based on the weight of the p-tert-butylphenol of sodium hydroxide. The so formed cycloalkanol is used in the '854 patent as an intermediate in the manufacture of organic sulfite esters.

Rowton et al, J. Org. Chem. *23* 1057 (1958) discloses the reaction of phenol with 1 - bromo - 2,3 - epoxybutane and 3 - bromo - 1,2 - epoxybutane to yield 3 - phenoxy - 1,2 - epoxybutane and 1 - phenoxy - 2,3 - epoxybutane, respectively. Both of these reactions do not occur in the presence of sodium.

Parker et al, Chem Rev. *59*, 737 (1959) is a review article discussing mechanisms of epoxide reactions. Although reactions employing cyclohexene oxide are mentioned, none of the reactions involving this compound include the reaction of cyclohexene oxide with compounds having the formula ROH, where R is phenyl or phenyl substituted.

Posner et al, JACS 99:25 8208 (1977) teaches the reaction of cycloalkene oxides with alumina in which allylic alcohols are the major product of this reaction. Cyclohexene oxide is specifically indicated to be unusual in that the major product of its reaction with alumina is the trans 1,2-diol. No disclosure is included of reacting those epoxides with a compound of the formula ROH.

Posner et al, JACS *99:25*, 8214 (1977), a related article, is directed to the same subject matter as the previously recited reference. This reference is further removed from the instant invention in that it is directed to the reaction of 3 epoxides: cyclopentadiene monooxide; 1,3-cyclohexadienemonooxide; and indene oxide, which are each acid-sensitive. These compounds are reacted with alumina to produce alcoholic products similar to those produced in the previously discussed reference.

The above discussion of the prior art establishes that the processes known for forming a compound having the formula R—O—R$^1$OH, where R is phenyl or phenyl substituted and R$^1$ is cycloalkylene or substituted cycloalkylene is limited in the above prior art to the disclosure of the reactionof 4-tert. butylphenol and cyclohexene oxide using sodium hydroxide at a concentration of 1 to 1.5% by weight, based on the weight of the substituted phenol. The difficulties of this process include the relatively large concentration of sodium hydroxide required. This not only imposes an economic penalty on the process but, in addition, necessitates neutralization of the product of the reaction with acid. The resultant requirement that the water of neutralization be removed further emphasizes the disadvantage of the prior art process. In the preferred embodiment wherein the product of this process is used in the preparation of the sulfite, failure to completely neutralize the reaction product results in the destruction of some of the other reactant, thionyl chloride or chlorosulfinate.

The prior art process is also deficient in that the purity of the product produced is of a degree that requires a further purification step.

E. Muller in "Methoden Der Organischen Chemie" (1956) discloses that sodium alcoholates are usuable as catalyst in the above reactions, however the specific reaction conditions are not discussed. Source material on which this discussion was based, however, reveals that the reactions occurred in methanol under reflux at about 65°C.

### Best summary of the invention

The instant invention is directed to a new process for preparing aromatic ethers useful as intermediates in the manufacture of organic sulfite esters which provide important improvements over the process of the prior art. In the process of this invention very small amounts of sodium containing catalyst is employed. This represents a cost savings over the process of the prior art in which relatively large amounts of sodium hydroxide are required. The process of the instant invention is also characterized by the absence of an acid environment in which the product of the reaction is formed. This not only eliminates the need to deal with an acid but, more importantly, eliminates the need for a neutralization step and the consequential need for a removal operation. Finally, the product of this process, compared to that produced in the prior art process, is produced in a purer form eliminating or at least significantly reducing purification operations.

In accordance with the instant invention there is provided a process for the synthesis of a compound having the formula R—O—R$^1$—OH, where R is phenyl or phenyl substituted with $C_1$—$C_4$ alkyl, $C_1$—$C_4$ alkoxy or methylenedioxy; and R$^1$ is $C_5$—$C_6$ cycloalk-1,2-ylene or $C_5$—$C_6$ cycloalk-1,2-ylene substituted with $C_1$—$C_4$ alkyl or $C_1$—$C_4$ alkoxy characterised by reacting a compound having the formula ROH, where R has the meanings above, with a compound selected from cyclopentene oxide, cyclohexene oxide, or cyclopentene oxide or cyclohexene oxide substituted

with $C_1$—$C_4$ alkyl, $C_1$—$C_4$ alkoxy or vinyl in the presence of a catalytically effective amount of a sodium containing agent selected from sodium metal, $C_1$—$C_8$ sodium alkoxide, $C_6$—$C_{10}$ sodium aryloxide, $C_7$—$C_9$ sodium alkaryl-oxide and sodium hydride at a temperature between 170° and 225°C.

## Detailed description

The compounds prepared according to the instant invention have the formula R—O—$R^1$OH, where R is phenyl or phenyl substituted with $C_1$—$C_4$ alkyl, $C_1$—$C_4$ alkoxy or methylenedioxy; and $R^1$ is $C_5$—$C_6$ cycloalk-1,2-ylene or $C_5$—$C_6$ cycloalk-1,2-ylene substituted with $C_1$—$C_4$ alkyl, $C_1$—$C_4$ alkoxy. Compounds of this kind are useful in the manufacture of organic sulfite esters. These organic sulfite esters are useful as pesticides and especially as miticides.

In the process of the instant invention, a compound having the formula ROH, where R has the meanings given above, is reacted with a compound selected from the group consisting of cyclopentene oxide, cyclohexene oxide, or either of cyclopentene oxide or cyclohexene oxide substituted with $C_1$—$C_4$ alkyl, $C_1$—$C_4$ alkoxy or vinyl. In the process of this invention, the above-recited reaction occurs in the presence of a catalytically effective amount of sodium in the form of sodium metal, the selected sodium oxides as defined in the claims or sodium hydride.

In a preferred embodiment of the process of this invention, stoichiometric amounts of the two reactants or up to a 20% molar excess of the oxide are used. The amount of sodium catalyst necessary to effectively catalyze the reaction is from 0.01 to 5% by weight, based on the weight of the compound, ROH. The reaction occurs over a period of from 15 minutes to 2 hours. The reaction occurs at a temperature in the range of between 170°C and 225°C.

More preferably, the amount of sodium catalyst employed in the reaction is from 0.05% to 1% by weight, based on the weight of the compound, ROH. In this more preferred embodiment, the reaction occurs at a temperature in the range of between 170°C and 210°C. The reaction time in the more preferred embodiment is from *15 mins.* to *2 hours.* In this more preferred embodiment the sodium agent is sodium metal. The following examples illustrate the process of this invention

## Example 1

15.0 g (0.1 mol) p-tert. butylphenol and 6.7 (0.29 mmol) of sodium metal were combined in a flask previously dried by sweeping with nitrogen gas. The two solids were heated to 210°C and maintained at this temperature while 9.8 g (0.1 mmol) of liquid cyclohexene oxide was added dropwise with stirring. The cyclohexene oxide was introduced over a period of 40 minutes during which time the color of the contents of the flask changed from pale yellow to nearly colorless. The mixture was heated for an additional 30 minutes at this temperature (210°) to complete the reaction. The product mixture was swept with nitrogen gas and heated under reduced pressure (10 mm Hg) to remove any unreacted cyclohexene oxide. The product obtained, 2 - (4 - tert. - butylphenoxy)-cyclohexanol, upon standing, crystallized. A yield of 22.8 g, equivalent to 92% of the theoretical of the product was obtained.

The product was subjected to a gas chromotography (GC) assay. The assay indicated 8.29% (2 - (4 - tert. - butylphenoxy)cyclohexanol; 10.1% unreacted p - tert. - butylphenol; and 7.0% of 2 - [2 - (4 - tert. - butylphenoxy)cylohexyloxy]cyclohexanol.

## Example 2

375.6 g (2.5 mols.) of p-tert.-butylphenol and 0.94 g (0.25 weight percent, based on the weight of p-tert.-butylphenol) of sodium metal was introduced into a 2-liter 3-necked reaction flask, equipped with a dry-ice condenser, mechanical stirrer, thermometer, 250 ml additional funnel and nitrogen inlet tube. The flask was heated under a blanket of nitrogen gas to melt the p-tert.-butylphenol. Most of the p-tert.-butylphenol was melted at 95°—100°C. The sodium metal reacted rapidly with the evolution of hydrogen gas. Thereafter, the contents of the flask was further heated and maintained at a temperature of 195°C—200°C. At this temperature, 265.6 ml (2. 63 mols) of cyclohexene oxide was added dropwise at a rate such that no cyclohexene oxide escaped as a vapor through the condenser. After this addition, the reaction mixture was further heated for an additional hour. It was then cooled to 120°C and transferred to 1 liter, round-bottom flask. The crude product was stripped at 140°C at a pressure of 0.1 to 1.0 mm Hg to remove excess cyclohexene oxide and any unreacted p-tert.-butylphenol. On cooling, the product was a colorless solid present in a yield of 624.8 g. The product was a colorless solid having a melting point of 86—90°C.

A GC assay was conducted on the product. The 2 - (4 - tert. - butylphenoxy)cyclohexanol represented 97.6% and p-tert.-butylphenol represented 0.27% of the product.

## Example 3

68,200 g (455 mols) of p-tert.-butylphenol was placed in a 75-gallon stainless steel reactor, equipped with an oil heating system. The reactor was exhausted to a high vacuum and then flushed with nitrogen gas. As a result of these steps, less than 0.25% of oxygen remained in the reactor. At this point the reactor was closed and heated to 130 to 140°C to melt the p-tert-butylphenol. While agitating, the reactor was cooled to 104—110°C. Upon reaching this temperature, 170.25 g of sodium metal was added. This addition occurred in three steps. During the first step a slow stream of nitrogen gas was introduced and the reactor vented through a steam-traced pipe. After each addition of sodium, an exotherm (3 to 4°C) was observed. Upon completion of the sodium addition, nitrogen flow was cut off and the reactor closed. The reactor was thereupon heated to a temperature of approximately 195.2C. 47,670 g (486 mols) of cyclohexene oxide was then added to the reactor at a rate sufficient to maintain the temperature at about 195°C and at a pressure of not more than 5 psig. The addition of cyclohexene oxide was completed in three hours. For an additional two hours, the reactor, with its contents, was maintained at 195°C. Thereupon, the reaction mixture was cooled to 150°C and stripped under a reduced pressure (7.0 mm Hg) at 150°C to remove excess cyclohexene oxide and any unreacted p-tert.-butylphenol.

A sample of the product was analyzed by gas chromatography. This assay resulted in the determination that the product represented 94.4% of 2 - (4 - tert. - butylphenoxy)cyclohexanol. No unreacted p-tert.-butylphenol was discovered in the assay of the product.

Example 4

Employing the procedure of Example 1, 75.1 g (0.5 mol) of p-tert.-butylphenol was introduced with 0.12 g of sodium metal (0.15 weight percent, based on the weight of the p-tert.-butylphenol). Again, using the procedure of Example 1, 53 ml (0.525 mol) of cyclohexene oxide was added to the reaction mixture. A crude product yield of 115.7 g (93.3%) was obtained. A GC assay indicated that the product was 89.5% 2 - (4 - tert. - butylphenoxy)cyclohexanol and 3.2% p-tert-butylphenol.

Example 5

The procedure of Example 2 was repeated. Thus, the exact same amount of p-tert.-butylphenol, 375.6 g (2.5 mols) was again introduced into the 2-liter flask. However, instead of employing sodium metal, the catalyst of this example was sodium methoxide. The sodium methoxide was introduced as a 25 weight percent solution in methanol. The solution possessed a specific gravity of 0.945 and 10 ml of this soluion was introduced dropwise into the flask. The reaction was thereafter conducted in accordance with the procedure of Example 2.

A crude produce yield of 622.3 grams was obtained. A GC assay of the product indicated that it represented 96% 2 - (4 - tert. - butylphenoxy)-cyclohexanol. The GC assay furthermore indicated the absence of unreacted p-tert.-butylphenol.

Claims

1. A process for making a compound having the formula ROR$^1$OH, where R is phenyl or phenyl substituted with $C_1$—$C_4$ alkyl, $C_1$—$C_4$ alkoxy or methylene dioxy; and R$^1$ is $C_5$—$C_6$ cycloalk-1,2-ylene or $C_5$—$C_6$ cycloalk-1,2-ylene substituted with $C_1$—$C_4$ alkyl or $C_1$—$C_4$ alkoxy characterised in that the process comprises reacting a compound having the formula ROH, where R has the meanings above, with a compound selected from cyclopentene oxide, cyclohexene oxide, cyclopentene oxide substituted with $C_1$—$C_4$ alkyl, $C_1$—$C_4$ alkoxy or vinyl and cyclohexene oxide substituted with $C_1$—$C_4$ alkyl, $C_1$—$C_4$ alkoxy or vinyl in the presence of a catalytically effective amount of a sodium containing agent selected from sodium metal, $C_1$—$C_8$ sodium alkoxide, $C_6$—$C_{10}$ sodium aryloxide, $C_7$—$C_9$ sodium alkaryloxide and sodium hydride, at a temperature between 170° and 225°C.

2. A process in accordance with Claim 1 characterised in that the sodium agent is present in a concentration of 0.05% to 1% by weight, based on the weight of the ROH.

3. A process in accordance with Claim 1, wherein the amount of sodium containing agent is in the range of between 0.01% and 5% by weight, based on the weight of the ROH.

4. A process in accordance with Claim 3 characterised in that the reaction occurs at a temperature in the range of between 170° and 210°C over a time period of between 15 minutes and 2 hours.

5. A process in accordance with any of the preceding claims, characterised in that the sodium containing agent is selected from sodium metal, sodium methoxide, sodium ethoxide, sodium isopropoxide, sodiumphenoxide and sodium p-tolueneoxide.

6. A process for preparing 2 - (4 - tert. - butylphenoxy)cyclohexanol, characterised in that the process comprises reacting 4-tert.-butylphenol with cyclohexene oxide in the presence of a catalytic amount of sodium metal at a temperature between 170° and 225°C.

7. A process in accordance with Claim 6 characterised in that the sodium metal is present in a concentration of from 0.01 to 1% by weight, based on the weight of the p-tert.-butylphenol.

8. A process in accordance with Claim 6, characterised in that the reaction occurs over a period of 5 minutes to 6 hours.

Patentansprüche

1. Ein Verfahren zum Herstellen einer Verbindung der Formel ROR$^1$OH, worin R Phenyl oder Phenyl substituiert mit $C_1$—$C_4$ Alkyl, $C_1$—$C_4$ Alkoxy oder Methylendioxy ist; und R$^1$ $C_5$—$C_6$ Cycloalk-1,2-ylen oder $C_5$—$C_6$ Cycloalk-1,2-ylen substituiert mit $C_1$—$C_4$ Alkyl oder $C_1$—$C_4$ Alkoxy ist, dadurch gekennzeichnet, daß das Verfahren das Umsetzen einer Verbindung der Formel ROH, worin R die obigen Bedeutugen hat, mit einer Verbindung ausgewählt aus Cyclopentenoxid, Cyclohexenoxid, Cyclopentenoxid substituiert mit $C_1$—$C_4$ Alkyl, $C_1$—$C_4$ Alkoxy oder Vinyl und Cyclohexenoxid substituiert mit $C_1$—$C_4$ Alkyl, $C_1$—$C_4$ Alkoxy oder Vinyl in Gegenwart einer katalytisch wirksamen Menge eines Natrium enthaltenden Reagens, das aus Natriummetall, $C_1$—$C_8$ Natriumalkoxid, $C_6$—$C_{10}$ Natriumaryloxid, $C_7$—$C_9$ Natriumalkaryloxid und Natriumhydrid ausgewählt ist, bei einer Temperatur zwischen 170 und 225°C, umfaßt.

2. Ein Verfahren gemäß Anspruch 1, dadurch gekennzeichnet, daß das Natriumreagens in einer Konzentration von 0,05% bis 1 Gew.-%, bezogen auf das Gewicht des ROH, verliegt.

3. Ein Verfahren gemäß Anspruch 1, bei dem die Menge des Natrium enthaltenden Reagens im Bereich von 0,01% und 5 Gew.-%, bezogen auf das Gewicht des ROH, vorliegt.

4. Ein Verfahren gemäß Anspruch 3, dadurch gekennzeichnet, daß die Reaktion bei einer Temperatur im Bereich von 170 bis 210°C während einer Zeitspanne von 15 Minuten bis 2 Stunden abläuft.

5. Ein Verfahren gemäß einem der vorhergehenden Ansprüche, dadurch gekennzeichnet, daß das Natrium enthaltende Reagens aus Natriummetall, Natriummethoxid, Natriumethoxid, Natriumisopropoxid, Natriumphenoxid und Natrium-p-toluoloxid ausgewählt ist.

6. Ein Verfahren zum Herstellen von 2-(4-tert-Butylphenoxy)-cyclohexanol, dadurch gekennzeichnet, daß das Verfahren das Umsetzen von 4-tert.-4-Butylphenol mit Cyclohexenoxid in der Gegenwart einer katalytischen Menge von Natriummetall bei einer Temperatur zwischen 170 und 225°C umfaßt.

7. Ein Verfahren gemäß Anspruch 6, dadurch gekennzeichnet, daß das Natriummetall in einer Konzentration von 0,01 bis 1 Gew.-% bezogen auf das Gewicht des p-tert.-Butylphenol, vorliegt.

8. Ein Verfahren nach Anspruch 6, dadurch gekennzeichnet, daß die Reaktion während einer Zeitspanne von 5 Minuten bis 6 Stunden abläuft.

## Revendications

1. Procédé de préparation d'un composé ayant pour formule $ROR^1OH$, où R est phényle ou phényle substitué par alkyle $C_1$—$C_4$, alcoxy $C_1$—$C_4$ ou méthylène dioxy; et $R^1$ est cycloalk-1,2-ylène $C_5$—$C_6$ ou cycloalk-1,2-ylène $C_5$—$C_6$ substitué par alkyle $C_1$—$C_4$ ou alcoxy $C_1$—$C_4$, caractérisé en ce que le procédé comprend la réaction d'un composé ayant pour formule ROH, où R a les significations ci-dessus, avec un composé choisi parmi l'oxyde de cyclopentène, l'oxyde de cyclohexène, l'oxyde de cyclopentène substitué par alkyle $C_1$—$C_4$, alcoxy $C_1$—$C_4$ ou vinyle et oxyde de cyclohexène substitué par alkyl $C_1$—$C_4$, alcoxy $C_1$—$C_4$ ou vinyle, en présence d'une quantité catalytiquement efficace d'un agent contenant du sodium choisi parmi le sodium métallique, l'alcoolate de sodium $C_1$—$C_8$, l'aryloxide de sodium $C_6$—$C_{10}$, l'alkaryloxide de sodium $C_7$—$C_9$ et l'hydrure de sodium, à une température entre 170 et 225°C.

2. Procédé selon la revendication 1, caractérisé en ce que l'agent de sodium est présent à une concentration de 0,05% à 1% en poids, en se basant sur le poids de ROH.

3. Procédé selon la revendication 1, où la quantité de l'agent contenant du sodium est comprise entre 0,01% et 5% en poids, en se basant sur le poids de ROH.

4. Procédé selon la revendication 3, caractérisé en ce que la réaction se produit à une température comprise entre environ 170 et 210°C sur une période de temps comprise entre 15 minutes et 2 heures.

5. Procédé selon l'une quelconque des revendications précédentes, caractérisé en ce que l'agent contenant du sodium est choisi parmi le sodium métallique, le méthoxyde de sodium, l'éthoxyde de sodium, l'isopropoxyde de sodium, le phénoxyde de sodium et le p-toluèneoxyde de sodium.

6. Procédé de préparation de 2-(4-tert.-butylphénoxy)cyclohexanol, caractérisé en ce que le procédé comprend la réaction du 4-tert.-4-butylphénol avec l'oxyde de cyclohexène en présence d'une quantité catalytique de sodium métallique à une température entre 170 et 225°C.

7. Procédé selon la revendication 6, caractérisé en ce que le sodium métallique est présente en une concentration de 0,01 à 1% en poids, en se basant sur le poids du p-tert.-butylphénol.

8. Procédé selon la revendication 6, caractérisé en ce que la réaction se produit sur une période de 5 minutes à 6 heures.